# FASCICULE DE BREVET EUROPEEN

(11) **EP 0 804 408 B1**
(45) Date de publication et mention de la délivrance du brevet: **05.09.2001**
(21) Numéro de dépôt: 95924395.7
(22) Date de dépôt: 30.06.1995
(51) Int. Cl.: C07C 69/88, A61K 31/25, C07C 67/08, C07C 235/46, C07C 233/31

(54) **NOUVEAUX COMPOSES 2,6-DI-TERT-BUTYLPHENOLS SUBSTITUES EN POSITION 4, LES COMPOSITIONS PHARMACEUTIQUES LES CONTENANT ET LEUR PROCEDE DE PREPARATION**
IN 4-POSITION SUBSTITUIERTE 2,6-DI-TERT-BUTYLPHENOLE, DIESE ENTHALTENDE PHARMAZEUTISCHE COMPOSITIONEN UND IHRE HERSTELLUNGSVERFAHREN
NOVEL 4-SUBSTITUTED 2,6-DI-TERT-BUTYLPHENOL COMPOUNDS, PHARMACEUTICAL COMPOSITIONS CONTAINING SAME AND PREPARATION METHOD THEREFOR

(30) Priorité: 30.06.1994 FR 9408061
(43) Date de publication de la demande: 05.11.1997
(73) Titulaire: FILECO S.A., 75009 Paris (FR)
(72) Inventeur: VACHY, Robert, F-75009 Paris (FR); WICHROWISKI, Boguslaw, F-92260 Fontenay-au-Rose (FR)
(74) Mandataire: Pernez, Helga
(86) Numéro de dépôt international: FR9500882
(87) Numéro de publication internationale: WO9600713

(56) Documents cités:
- EP-A- 0 200 685
- WO-A-92/08450
- US-A- 3 944 594
- US-A- 3 972 927
- US-A- 4 032 562

## Description

La présente Invention concerne de nouveaux composés 2,6-di-t-butylphénols substitués en position 4. L'Invention a aussi pour objet les compositions pharmaceutiques contenant ces composés et l'utilisation de ces derniers pour l'obtention de médicaments antiviraux, ainsi que la préparation de ces composés.

Des composés 2,6-di-t-butylphénols substitués en position 4, aussi dénommés selon une autre nomenclature 3,5-di-t-butyl-4-hydroxybenzènes substitués en position 1, sont connus de longue date. La préparation de certains d'entre-eux par oxydation du 2,6-di-t-butyl-4-méthylphénol a notamment été décrite par G. R. Yohe et al., dans *J. Org. Chem.* (1956), 21, 1289-1292. Du fait de leur propriété antioxydante ces composés ont initialement été utilisés dans les produits pétroliers, puis comme additifs alimentaires en raison de leur activité sur les graisses animales (J. C. Dacre, *Biochem. J.,* 1961, vol. 78, n°4, pp. 758-766).

Il a ensuite été montré que les composés 2,6-di-t-butylphénols substitués en position 4 par un reste hydrocarboné aliphatique, désignés BHT pour la dénomination anglaise "butylated hydroxytoluène", présentaient des propriétés antivirales à l'encontre des virus à enveloppes lipidiques (W. Snipes et al., *Science*, 1975, vol. 188, n° 4183, pp. 64-65), en conséquence, il a été proposé, par exemple dans la Demande de Brevet Français publiée sous le numéro 2 507 891 ou dans la dans la Demande de Brevet Internationale PCT/FR91/00882, d'utiliser ces composés pour l'obtention de médicaments destinés au traitement des maladies liées à une infection d'un individu par les virus, notamment les virus de l'herpès.

La toxicité du BHT a été discutée, notamment par J. G. Llaurodo (*West J. Med.,* 1983, vol. 139, n° 2, pp. 229-230), D. M. Shlian (*N. Engl. J. Med.,* 1986, vol. 314, n° 10, pp. 648-649) et M. W. Grogan (*West J. Med.,* 1986, vol. 145, n° 2, pp. 245-246), et de nouveaux dérivés 2,6-di-t-butylphénols ont été proposés notamment comme agents anti-allergiques dans la Demande de Brevet Européen publiée sous le numéro 0 212 848 et le Brevet des Etats-Unis d'Amérique n° 4 968 710.

L'acide 3,5-di-t-butyl-4-hydroxybenzoïque, dénommé aussi BG4, de même que ses halogénures, notamment les bromures et chlorures, ont par ailleurs été décrits dans la Demande de Brevet Européen publiée sous le numéro 0 269 981 et le Brevet des Etats-Unis d'Amérique n° 4 708 966 comme intermédiaires de synthèse. Il a été proposé dans la Demande de Brevet Internationale PCT/FR91/00882 d'utiliser cet acide, parmi d'autres composés 2,6-di-t-butylphénols substitués en position 4, pour préparer des médicaments antiviraux destinés au traitement des maladies liées à une infection d'un individu par les virus du type à enveloppe lipidique et plus particulièrement les virus de l'herpès, ou par les papillomavirus.

La Demande de Brevet Internationale PCT/FR91/00882 précitée décrit des composés 2,6-di-tert-butylphénols substitués en position 4 répondant à la formule suivante : 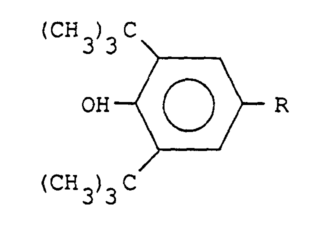 dans laquelle R est un groupe alkyle en C₁-C₁₂, un groupe alcényle en C₂-C₁₂, un groupe alcynyle en C₂-C₁₂, un groupe alcoxy en C₁-C₁₂, un groupe formyle, un groupe alkanoyle en C₂-C₁₂, un groupe hydroxyalkyle en C₁-C₁₂, un groupe amine primaire, secondaire ou tertiaire, un groupe -OCH₃, -CH₂OH, -CHO ou un groupe -COOH ou un groupe de formule : -A-COOH, dans lequel A est un reste hydrocarboné aliphatique en C₁-C11. Cette Demande de Brevet envisage aussi les sels et esters des composés précédents lorsque R est COOH ou A-COOH.

Les acides répondant à la formule précédente et notamment l'acide 3,5-di-t-butyl-4-hydroxybenzoïque, ci-après dénommé BG4,se sont avérés particulièrement intéressants pour l'obtention de médicaments destinés au traitement des sujets infectés par les virus de l'herpès ou les papillomavirus; en outre, d'une manière générale, ces acides sont présentés comme possédant une meilleure activité que leurs sels et esters.

Mais le problème majeur posé par ces acides réside dans leur mauvaise solubilité dans les solvants physiologiques aqueux habituels. Cet inconvénient ne s'oppose pas à l'utilisation de ces composés dans des préparations solides ou semi-liquides, tels que des gels, des crèmes ou pommades, lesquels sont particulièrement adaptés pour une application locale. Cependant, la mauvaise solubilité du BG4 pose un problème pour la préparation de solutions injectables par voie intraveineuse, mais aussi, lorsqu'il a s'agit de tester des solutions de ce produit in vitro sur des modèles cellulaires pour mettre en évidence de nouvelles activités de ce composé. Ce problème a été rencontré notamment lors de l'étude mené sur l'action virucide du BG4 sur le pouvoir infectieux du virus HIV-1 vis à vis de cellules cibles MT4 (S. Harada et al., *Science*, 1986, 229, 563-566 ; F. Rey et al., *J. of Virol. Meth*., 1987, 16, 239-249). Cette étude a donc été réalisée avec des cristaux micronisés du BG4 en suspension dans un tampon; la solubilité du BG4 dans le DMSO n'a pas été utilisée du fait de l'effet toxique du DMSO sur les cellules; de même les esters ou les sels de BG4 n'ont pas été utilisés en raison de la hausse du pH, néfaste aux cellules, qu'ils entraînent.

La Demanderesse, qui s'intéresse de longue date aux composés 2,6-di-t-butylphénols substitués en position 4 et notamment au BG4, a cherché à concevoir et préparer de nouveaux dérivés ne présentant pas les inconvénients des membres connus de cette famille de composés et possédant des propriétés pharmacologiques équivalentes ou supérieures.

Les travaux de recherche qui ont été menés par la Demanderesse ont conduit à une nouvelle série de dérivés, esters et amides, des composés 2,6-di-t-butylphénols substitués en position 4 par une fonction acide (-COOH) ou par un reste hydrocarboné aliphatique portant une telle fonction acide.

Les composés de l'Invention répondent à la formule générale: 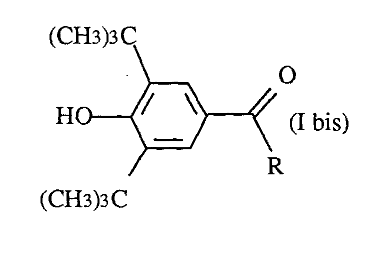 dans laquelle le radical R est choisi parmi les 3 groupements de formules suivantes :

― (OCH₂CH₂)ₙ―Y (II)

― O(CH₂CH₂NH)ₙ―Y (III)

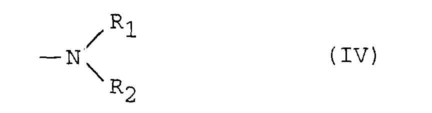

dans lesquelles :
. n est un nombre entier compris entre 1 et 20; mais on peut tout à fait envisager une valeur maximale de n de 80 ;
. R1 est choisi parmi les groupes suivants:
   - (CH₂CH₂O)ₙ-Y, -(CH₂CH₂NH)ₙ-Y ;
. R2 est un atome d'hydrogène ou a la même signification que R1 ; et
. Y choisi parmi les groupes suivants: un atome d'hydrogène, un groupe -OH, un groupe -NH2, un groupe -COOH, un groupe 3,5-di-t-butyl-4-hydroxybenzoate, un groupe amine ou hydroxyamine primaire, secondaire ou tertiaire, un groupe amide, diamide, hydroxyamide ou halogénoamide aliphatique ou aromatique, un groupe alkoyle en C₁-C₁₂, un groupe glycoyle, un groupe glycéryle mono-, di- ou tri-substitué, un groupe hétérocyclique, un groupe thioester aliphatique ou aromatique, un groupe de formule : -COOZ, où Z est un groupe alkyle en C₁-C₁₂, un groupe alcényle en C₁-C₁₂, un groupe alcynyle en C₁-C₁₂, un groupe hydroxyalkyle en C₁-C₁₂, un groupe hydroxyalcynyle en C₁-C₁₂, un groupe aminoalkyle en C₁-C₁₂, un groupe aminoalcényle en C₁-C₁₂, un groupe aryl, un groupe hétérocyclique.

Conformément à la formule (I bis), on peut distinguer parmi les composés de l'Invention les esters et amides de l'acide 3,5-di-t-butyl-4-hydroxybenzoïque (BG4).

Parmi les esters, on peut citer plus particulièrement ceux de formule (I bis) dans laquelle R répond à la formule :

―(OCH₂CH₂)ₙ―Y (II)

Ces nouveaux esters selon l'Invention résultent de la réaction d'un composé 2,6-di-t-butylphénol substitué en position 4 par un groupe -COOH avec des polyooxyéthylènes glycols de formule générale :

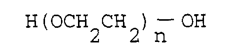

Les composés 2,6-di-tert-butylphénols résultants de cette réaction d'estérification conservent le groupe OH libre d'un glycol, lequel est susceptible de réagir avec une très grande variété de composés par estérification, deshydratation, oxydation, déshydrogénation. C'est précisément cette grande variété de réaction possible avec le groupe -OH libre de ces composés qui autorise à envisager une grande diversité de significations pour le groupe Y des formules (II), (III) et (IV).

La chaîne polyoxyéthylènique -(OCH₂CH₂)ₙ- confère à ces composés de l'Invention des propriétés tout à fait originales d'un point de vue physico-chimique. En effet, cette chaîne confère une action tensio-active qui favorise considérablement la solubilité des composés de l'Invention par rapport aux composés 2,6-di-tert-butylphénols substitués en position 4 connus dans l'Art antérieur.

Cette solubilité rend ces composés miscibles à l'eau en toute quantité et, en conséquence, autorise maintenant les applications nécessitant cette présentation. A la différence des esters des composés 2,6-di-tert-butylphénols substitués en position 4 par une fonction acide connus dans l'Art antérieur, les esters de l'Invention n'entraînent pas de modification de la valeur du pH des solutions qui les contiennent.

En outre, la valeur de n ne modifie pas les propriétés de solubilités de ces composés et l'on peut envisager des polymères de très longue dimension si n est de l'ordre de 80.

Un composé préféré de l'Invention est le 3,5-di-t-butyl-4-hydroxybenzoate d'octaoxyéthylèneglycol correspondant au composé de formule (I bis) dans laquelle Y représente un groupe OH et qui répond à la formule suivante :

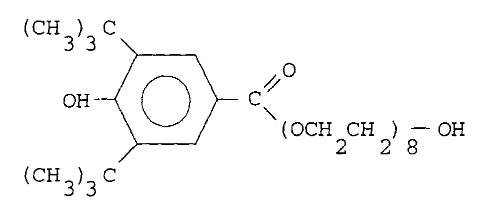

D'autres composés préférés de l'invention sont les composés de formule (I bis) pour lesquels, dans les formules (II), (III) et (IV), Y représente un groupe de formule :

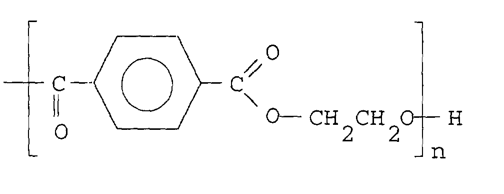

Les composés de l'Invention conservent les propriétés antivirales, dues au groupe 2,6-di-tert-butylphénol, des composés de l'Art antérieur. En outre, la chaîne représentée dans la formule (I bis) par le radical R, et notamment une chaîne polyoxyéthylènique, en raison de son effet sur la solubilisation des phospholipides membranaires, favorise la pénétration dans les virus des composés 2,6-di-tert-butylphénols substitués en position 4, et explique l'augmentation de l'activité antivirale des composés de l'Invention par rapport aux composés de la même famille connus dans l'Art antérieur. La Demanderesse a mis en évidence l'existence de ces propriétés avantageuses chez les composés de formule (I) ci-dessous.

L'Invention a pour objet les compositions pharmaceutiques caractérisées en ce qu'elles renferment au moins un composé de formule (I bis) éventuellement en association avec un véhicule pharmaceutique acceptable.

L'Invention a donc aussi pour objet les compositions pharmaceutiques renfermant à titre de principe actif au moins un composé de formule (I), 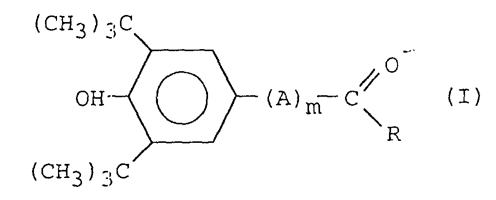 dans laquelle :
- A est un groupe -CH₂-,
- m est un nombre entier compris entre 0 et 12, et
- le radical R est choisi parmi les trois groupements de formules :

   ―(OCH₂CH₂)ₙ―Y (II)

   ―O(CH₂CH₂NH)ₙ―Y (III)

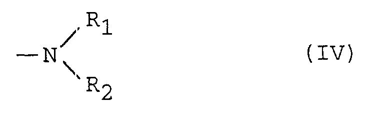

   dans lesquelles, n, R1, R2 et Y ont la même signification que dans la formule (I bis) ;
éventuellement en association avec un véhicule pharmaceutiquement acceptable habituellement utilisé.

Parmi les principes actifs entrant dans les compositions pharmaceutiques de l'Invention, il faut citer plus particulièrement les composés de formule (I) dans laquelle R représente un groupe de formule (II) et tout particulièrement le 3,5-di-t-butyl-4-hydroxybenzoate d'octaoxyéthylèneglycol.

Les compositions pharmaceutiques selon l'Invention peuvent être administrées de manière usuelle à l'homme par voie entérale ou parentérale, notamment par voie orale. Elles peuvent être sous la forme de préparations solides, semi-solides ou semi-liquides et liquides. Comme exemple on peut citer les comprimés, les gélules et dragées, les suppositoires, les gels, les pommades et les crèmes, les solutions ou les suspensions injectables, les gouttes et les sirops.

Compte-tenu de la bonne solubilité des composés de l'Invention dans les solvants physiologiques habituels et notamment les véhicules aqueux, ces composés peuvent, à la différence des composés 2,6-di-tert-butylphénols substitués en position 4 de l'Art antérieur, être facilement administrés par injection intraveineuse ou intramusculaire.

Mais bien entendu, les préparations solides, semi-solides et notamment les gels et les pommades demeurent parfaitement adaptées pour une application locale sur la peau ou au niveau des organes anogénitaux, lesquels sont plus particulièrement impliqués dans le processus de transmission des maladies sexuellement transmissibles, particulièrement les maladies virales liés à une infection par les virus de l'herpès, les virus HIV et les papillomavirus.

Dans ces compositions le principe actif est généralement mélangé avec un ou plusieurs excipients pharmaceutiquement acceptables habituels bien connus de l'Homme du métier.

Les compositions pharmaceutiques peuvent contenir notamment de 0,1 à 20% en poids de principe actif constitué d'au moins un composé de formule I.

La quantité de principe actif qui est administrée dépend évidemment du patient qui est traité, de la voie d'administration et éventuellement de l'avancement de la maladie.

Dans ces compositions, le composé de l'Invention peut être associé à d'autres agents actifs connu de l'Homme du métier pour leur synergie avec les composés 2,6-di-tert-butylphénols substitués en position 4, comme le propolis.

Compte-tenu des propriétés anti-virales des composés 2,6-di-tert-butylphénols substitués en position 4, les composés de formule (I) peuvent être aussi avantageusement utilisés pour l'obtention d'un médicament destiné au traitement et à la prévention chez l'homme et l'animal d'une infection par les virus. Plus particulièrement les composés de l'Invention sont utiles pour l'obtention d'un médicament destiné au traitement et à la prévention d'une infection par les virus à enveloppe lipidique, comme les virus de l'herpès ou les virus du type HIV. Ils sont aussi utiles pour l'obtention d'un médicament destiné au traitement et à la prévention d'une infection par les papillomavirus, par exemple pour le traitement des condylomes notamment ano-génitaux, dûs aux papillomavirus humains.

La Demanderesse a en outre mis en évidence le rôle des composés de L'Invention, et plus particulièrement du 3,5-di-t-butyl-4-hydroxybenzoate d'octaoxyéthylèneglycol, lorsqu'ils sont associés à un inhibiteur de la NO-synthase inductible comme le L-NMMA, pour traiter les maladies dans lesquelles le monoxyde d'azote à une action pathologique, tels que l'inflammation, la constriction vasculaire ou les cancers.

Cette propriété est donc particulièrement intéressante, car l'effet anti-viral des composés de l'Invention et notamment du 3,5-di-t-butyl-4-hydroxybenzoate d'octaoxyéthylèneglycol, utiles dans le traitement du SIDA, est renforcée par les propriétés anti-inflammatoires de ces composés sur la réplication des virus HIV.

L'Invention concerne donc également l'utilisation des composés de formule (I) en association avec un inhibiteur de la NO-synthase industible comme le L-NMMA., pour l'obtention d'un médicament destiné au traitement des maladies dans lesquelles le monoxyde d'azote à une action pathologique.

Par ailleurs, les composés de l'Invention et plus particulièrement ceux de formule (I) dans laquelle Y est un groupe OH, sont susceptibles de réagir, par exemple par estérification, avec une grande variété de produit. Les caractéristiques physico-chimiques des composés de l'Invention et notamment cette réactivité particulière, couplée à l'activité antivirale du reste 2,6-di-tert-butylphénol permet d'envisager des applications très originales dans le domaine des tissus pharmaceutiques tels que les pansements, bandages et compresses et autres d'implants commes les fils, sutures, prothèses, pach, pour lesquels les composés de l'Invention peuvent être utilisés pour renforcer les propriétés antiseptiques, antivirales, antibactériennes et antifongiques. Les composés de l'Invention peuvent, en effet, être greffés sur une partie au moins des fibres constituant ledit tissu pharmaceutique ou l'implant.

L'Invention concerne donc aussi un tissu pharmaceutique du type pansement, bandage ou compresse ou implant, caractérisé en ce qu'au moins un composé de formule (I) est greffé sur tout ou partie des fibres constituant ledit tissu pharmaceutique ou l'implant.

A titre d'exemple d'une telle application, on peut citer le greffage du 3,5-di-t-butyl-4-hydroxybenzoate d'octaoxyéthylèneglycol sur le Dacron (marque déposée qui désigne une fibre textile synthétique de polyester) ou le terylène, lesquels entrent dans la composition de nombreux tissus pharmaceutiques ou d'implants.

Le Dacron répond à la formule suivante : 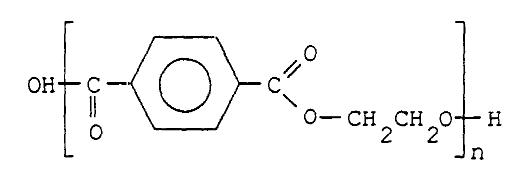 et peut donc réagir via la fonction acide carboxylique teminal par estérification avec le groupe OH du 3,5-di-t-butyl-4-hydroxybenzoate d'octa-oxy-éthylèneglycol, pour conduire au composé suivant :

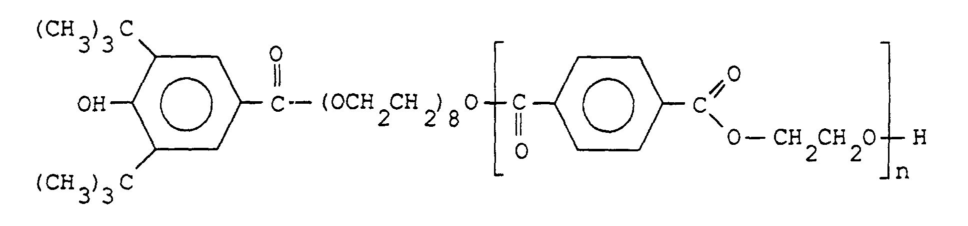

La réactivité du groupe OH de la fonction acide carboxylique terminal du Dacron est bien connue; elle est notamment utilisée pour la préparation du terylène dont la formule est la suivante :

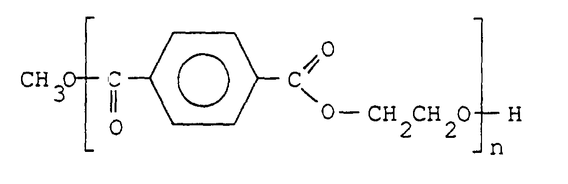

Les composés de l'Invention peuvent être préparés selon des procédés connus par estérification ou amidation de composés 2,6-di-t-butylphénols substitués en position 4 par un groupe -COOH ou par un reste hydrocarboné aliphatique portant un groupe -COOH.

En ce qui concerne l'estérification notamment de l'acide 3,5-di-tert-butyl-4-hydroxy-benzoïque, celle-ci est réalisée avec des alcools de formules : H(OCH₂CH₂)ₙ-OH ou HO(CH₂CH₂NH)ₙ-OH.

Les composés résultant de cette réaction d'estérification conservant leur second groupe -OH libre, ils sont susceptibles de réagir avec une très grande variété de composés notamment par estérification, deshydratation, oxydation, déshydrogénation.

Mais l'on peut également envisager de réaliser la première réaction d'estérification directement avec les alcools précédents portant le groupement Y et répondant alors aux formules : H(OCH₂CH₂)ₙ-Y ou HO(CH₂CH₂NH)ₙ-Y.

L'Invention a donc aussi pour objet les procédés de préparation des esters de formule (I) comprenant :
- une première étape d'estérification d'un composé de formule : 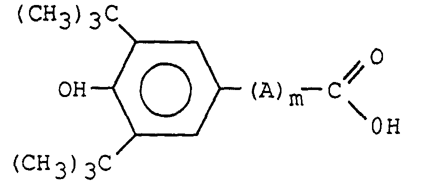 dans laquelle A et m ont la même signification que précédemment,
   par un composé de formules : H(OCH₂CH₂)ₙ-OH, ou HO(CH₂CH₂NH)ₙ-OH, dans lesquelles n à la même signification que dans la formule (I);
- éventuellement, une seconde étape d'estérification, de deshydratation, d'oxydation ou de déshydrogénation du composé obtenu à la première étape et répondant à l'une des formules suivantes : 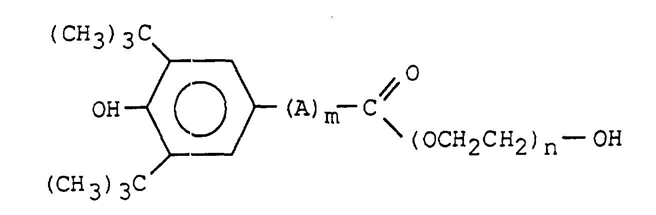 ou 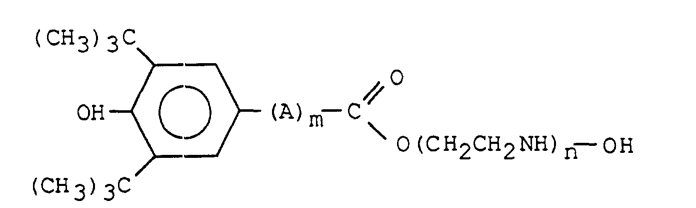 par un composé portant le groupement Y défini dans les formules (II), (III) et (IV).

Un procédé équivalent au précédent consiste à réaliser uniquement la première étape d'estérification d'un composé de formule : 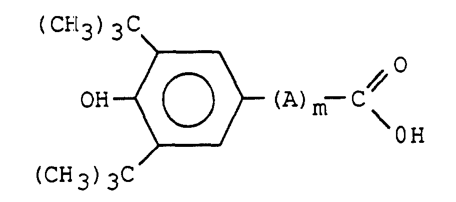 par un composé de formule : H(OCH₂CH₂)ₙ-Y ou HO(CH₂CH₂NH)ₙ-Y,

En ce qui concerne l'amidation notamment de l'acide 3,5-di-tert-butyl-4-hydroxy-benzoïque, celle-ci est réalisée avec des amines primaires ou secondaires de formules : 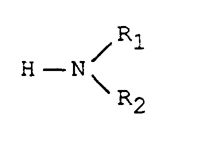 dans laquelle les radicaux R1 et R2 ont la même signification que dans la formule (IV).

Il est donné ci-après des exemples non limitatifs destinés à illustrer d'autres caractéristiques et avantages des composés de la présente Invention.

### I - PRAPARATION DE COMPOSÉS DE FORMULE (I).

### Exemple 1 : Préparation d'un ester de formule (I) dans laquelle Y est un groupe OH.

### a) Schéma réactionnel.

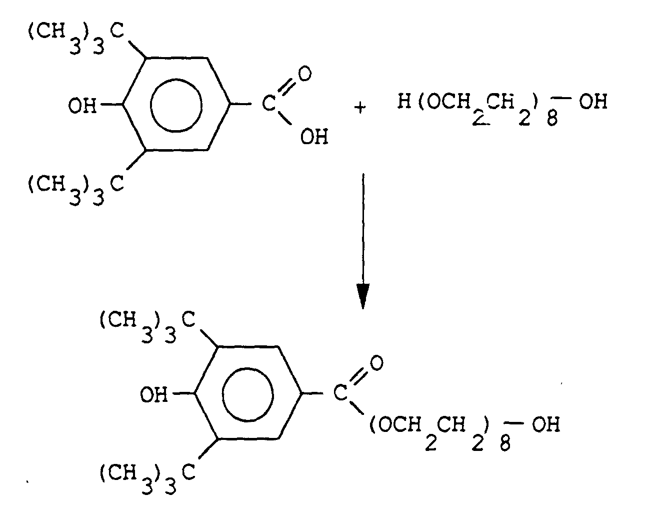

### b) Préparation des réactifs.

L'octaoxyéthylène glycol est séché par une distillation azéotropique en présence de benzène. Après séchage, il est utilisé immédiatement dans la synthèse ci-après.

### c) Synthèse du 3,5-di-t-butyl-4-hydroxybenzoate d'octaoxyéthylèneglycol.

Dans un ballon équipé d'un agitateur magnétique et d'un appareil de Dean Stark surmonté d'un réfrigérant à boules, on met :
- 300 ml de benzène sec,
- 22,5 g (90 mmoles) d'acide 3,5-di-t-butyl-4-hydroxybenzoïque (BG4),
- 50 g (135 mmoles) d'octaoxyéthylène glycol, et
- 1 ml d'acide sulfurique concentré.

Ce mélange est chauffé jusqu'à ébullition, au cours de laquelle on observe la dissolution du BG4, puis porté pendant 30 heures au reflux; le volume d'eau dégagé pendant la réaction est d'environ 1,6 ml.

Après refroidissement, le benzène est évaporé et le 3,5-di-t-butyl-4-hydroxy-benzoate d'octaoxyéthylèneglycol est purifié par chromatographie sur colonne.

### d) Purification par chromatographie sur colonne.

Conditions de purification :
- phase stationnaire : gel de silice;
- rapport (masse d'une phase stationnaire)/ (masse du mélange réactionnel sec) : 20;
- éluant : CHCl₃ puis le mélange CHCl₃/MeOH = 99/1, 98/2, 97/3, 96/4, 95/5;
- solvant de migration CHCl₃/MeOH = 90/10;
- révélateur : iode;
- Rf = 0,36.
Rendement final : 33,4 g (61,64%).
Formule brut du 3,5-di-t-butyl-4-hydroxybenzoate d'octaoxyéthylèneglycol : C₃₁H₅₄O₁₁ (PM : 602).

### Exemple 2 : Préparation d'un ester de formule (I) dans laquelle Y est un groupe 3,5-di-t-butyl-4-hydroxybenzoate.

### a) Schéma réactionnel.

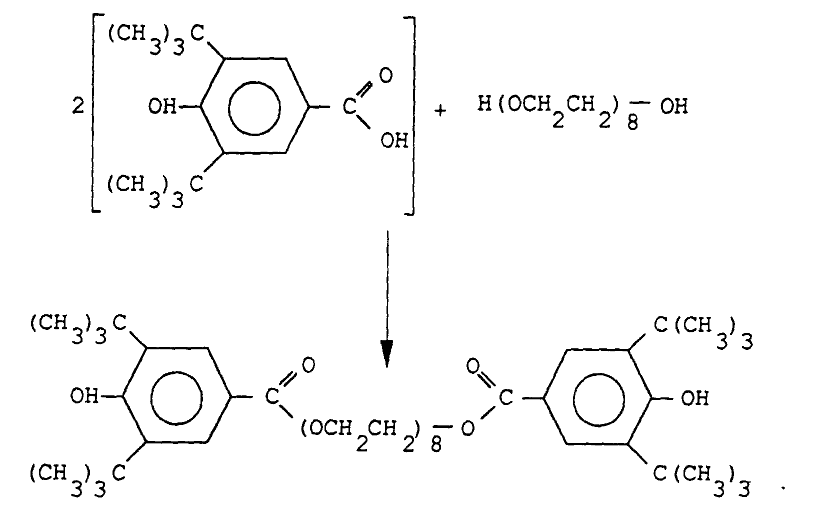

### b) Synthèse du α,ω-bis-(3,5-di-t-butyl-4-hydroxy-benzoyle)-octa-oxyéthylène.

Dans un ballon équipé d'un agitateur magnétique et d'un appareil de Dean Stark surmonté d'un réfrigérant à boules, on met :
- 300 ml de benzène sec,
- 22,5 g (90 mmoles) d'acide 3,5-di-t-butyl-4-hydroxy-benzoïque (BG4),
- 13,32 g (36 mmoles) d'octa-oxyéthylène glycol fraîchement séché, et
- 1 ml d'acide sulfurique concentré.

Ce mélange est chauffé sous reflux pendant 3 jours; le volume d'eau dégagé pendant la réaction est d'environ 1,3 ml.

Après refroidissement, le benzène est évaporé sous vide et le α,ω-bis-(3,5-di-t-butyl-4-hydroxy-benzoyle)-octa-oxyéthylène purifié par chromatographie sur colonne de gel de silice.

### Exemple 3 : Préparation d'un ester de formule (I) dans laquelle Y est un groupe amide aliphatique insaturé.

### a) Schéma réactionnel.

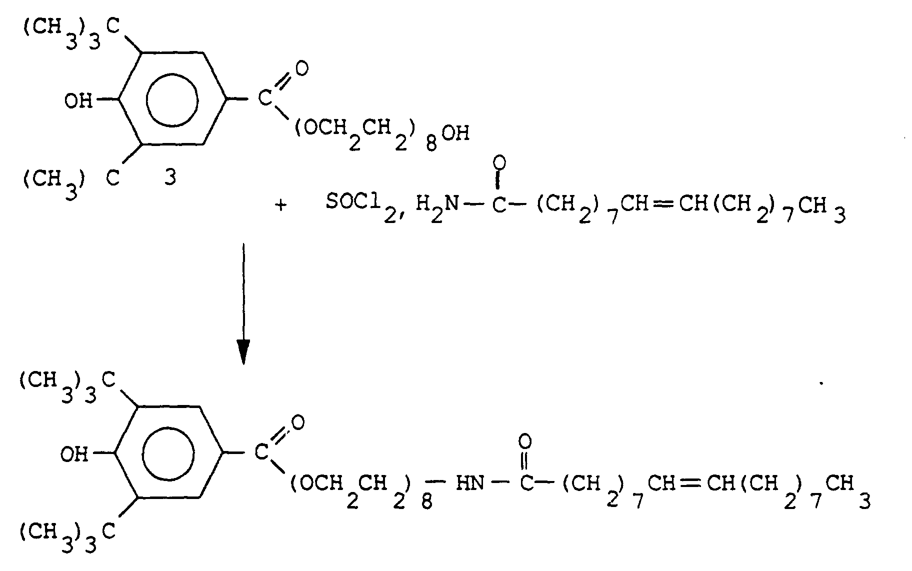

### b) Synthèse N-{α-(3,5-di-t-butyl-4-hydroxy-benzoile)-ω-chloro-octa-oxyéthylène}-oléique amide.

Dans un ballon équipé d'un réfrigérant, d'une ampoule à brome, d'un thermomètre et d'un agitateur, on met 24,08 g (40 mmoles de 3,5-di-t-butyl-4-hydroxy-benzoate d'octa-oxyéthylène glycol.

Puis, on ajoute goutte-à-goutte 6 g (50 mmoles) de chlorure de thionyle en agitant vigoureusement et en maintenant la température entre 5 et 10°C. Après l'addition complète, on chauffe au bain marie pendant 2 heures. On ajoute alors 150 ml de benzène et l'on décompose l'excès de chlorure de thionyle avec de l'eau.

Les phases étant séparées, on lave la phase aqueuse plusieurs fois avec de l'éther de pétrole. Les phases organiques réunies sont neutralisées avec une petite quantité de solution aqueuse de bicarbonate de sodium et séchées avec MgSO₄. Les solvants sont évaporés sous vide et le résidu est solubilisé dans 150 ml de benzène sec.

On ajoute alors goutte-à-goutte la solution de 22,48 g (80 mmoles) de α-(3,5-di-t-butyl-4-hydroxybenzoile)-ω-chloro-octa-oxyéthylène dans une solution de 22,48 g (80 mmoles) d'oléique amide dans 100 ml de benzène sec.

Le mélange réactionnel est chauffé sous reflux pendant 4 heures. Après refroidissement, on le neutralise avec une petite quantité de solution aqueuse de bicarbonate de sodium.

La phase organique est séparée et séchée avec MgSO₄. Le benzène est ensuite évaporé sous vide et le N-{α-(3,5-di-t-butyl-4-hydroxy-benzoile)-ω-chloro-octa-oxyéthylène}-oléique amide est séparé par chromatographie sur colonne de gel de silice.

### Exemple 4 : Préparation d'un ester de formule (I) dans laquelle Y est un groupe aminoalkoyle.

### a) Schéma réactionnel.

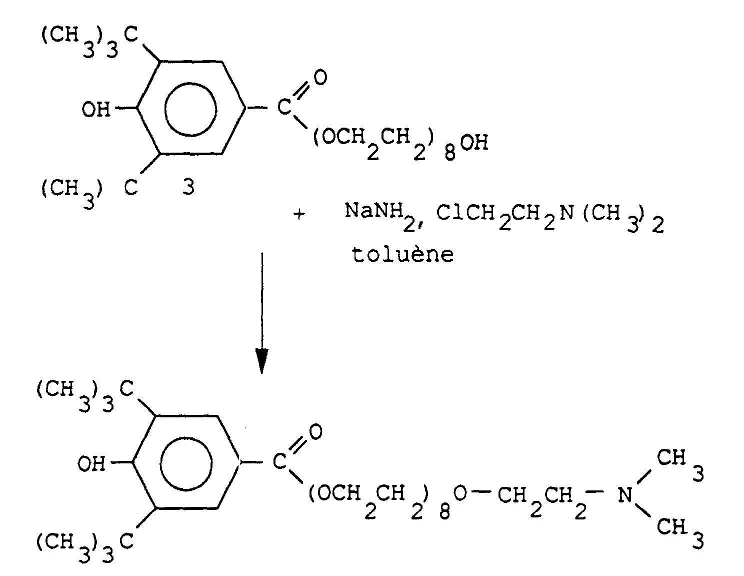

### b) Synthèse du α-(3,5-di-t-butyl-4-hydroxybenzoyle)-ω-(β'-N,N diméthylaminoéthyl) octa-oxyéthylène éther.

Dans un ballon muni de réfrigérant, d'une ampoule à brome, d'un thermomètre et d'un agitateur magnétique, on met l'amide de sodium fraîchement préparé à partir de 1,2 g (52 mmoles) de sodium en suspension dans 200 ml de toluène. On ajoute goutte-à-goutte pendant 2 heures une solution de 30,1 g (50 mmoles) de 3,5-di-t-butyl-4-hydroxybenzoate d'octa-oxy-éthylène glycol dans 300 ml de touluène sec. La température du mélange réactionnel doit être maintenue en dessous de 25°C.

Un fois l'addition terminée, on chauffe le mélange au bain marie pendant 6 heures. On ajoute alors 9 g (83,7 mmoles) de β-N,N-diméthylamino-α-chloroéthylène et on chauffe au bain marie pendant encore 18 heures. On décompose l'excès d'amide de sodium en ajoutant 100 ml d'eau.

Après refroidissement, on sépare la phase organique et la phase aqueuse est lavée plusieurs fois avec l'ether de pétrole. Après séchage avec MgSO₄ des phases organiques réunies, on évapore les solvants sous vide.

Le α-(3,5-di-t-butyl-4-hydroxy-benzoyle)-ω-(β'-N,N-diméthylaminoéthyl)octa-oxyéthylène éther, aussi dénommé N,N-diméthyl-2-{α-hydro-ω-(3, 5-di-t-butyl-4-hydroxy-benzoyle)octa-oxyéthylène}-éthanamine est séparé par chromatographie sur colonne de gel de silice.

### Exemple 5 : Préparation d'une amide de formule (I).

### a) Schéma réactionnel.

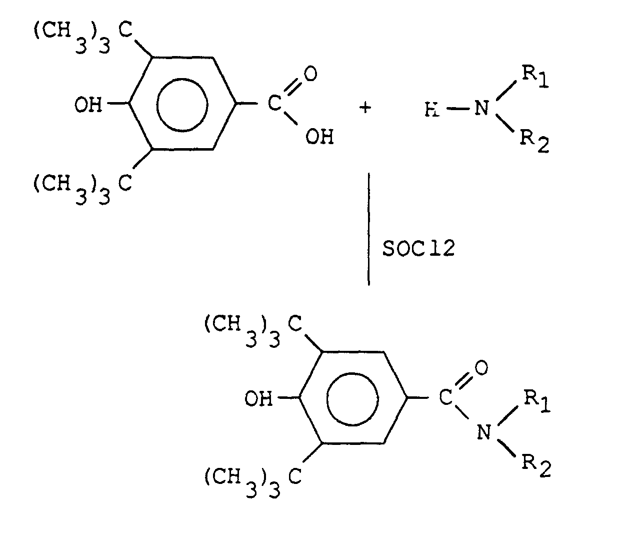

### b) Protocole expérimental.

Dans un ballon équipé d'un réfrigérant, d'une ampoule à brome, d'un thermomètre et d'un agitateur, on met 20 g (80 mmoles) d'acides 3,5-di-t-butyl-4-hydroxybenzoïque. On ajoute goutte-à-goutte 12 g (10 mmoles) de chlorure de thionyle en agitant vigoureusement et en maintenant la température en 5 et 10°C. Après l'addition complète, on chauffe au bain marie pendant 2 heures. On ajoute ensuite 150 ml de benzène et on décompose l'excès de chlorure de thionyle avec de l'eau.

Les phases étant séparées, on lave la phase aqueuse plusieurs fois avec de l'ether de pétrole. Les phases organiques réunies sont neutralisées avec une petite quantité de solution aqueuse de bicarbonate de sodium et séchées avec MgSO₄. Les solvants sont évaporés sous vide et le résidu est solubilisé dans 150 ml de benzène sec.

On ajoute alors goutte-à-goutte la solution benzénique de chlorure de 3,5-di-tert-butyl-4-hydroxybenzoyle dans une solution de 160 mmoles d'amine de formule ci-dessous dans 100 ml de benzène sec: 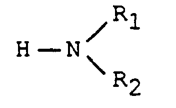 dans laquelle les radicaux R1 et R2 ont la même signification que dans la formule (IV).

Le mélange réactionnel est chauffé sous reflux pendant 4 heures. Après refroidissement, on le neutralise avec une petite quantité de solution aqueuse de bicarbonate de sodium. La phase organique étant séparée, elle est séchée avec MgSO₄; le benzène est ensuite évaporé sous vide. L'amide obtenu est séparé par chromatographie sur colonne de gel de silice.

### II - EXEMPLES DE FORMULATION DE COMPOSITION RENFERMANT UN COMPOSÉ DE FORMULE (I).

Il est donné ci-dessous des exemples non limitatifs de formulations du 3,5-di-t-butyl-4-hydroxybenzoate d'octaoxyéthylèneglycol.

### Crème dermique :

- CUTINA CBS :
   (commercialisé par la Société Henkel et constitué de : stéarate de glycérol, alcool cétostéarylique, palmitate de cétyle, triglycérides de coco). 7
- TEFOSE 1500 :
   (commercialisé par la Société Gattefosse et constitué de monodipalmitostéarate de polyoxyéthylène glycol). 10
- Stéarate d'isocétyle : 10
- Triglycérides en C₈ et C₁₀ (55/45) : 5
- Esters d'acide parahydroxybenzoïque : 0,25
- 3,5-di-t-butyl-4-hydroxybenzoate :
   d'octaoxyéthylèneglycol 2
- Eau déminéralisée : QSP 100 g

### Gel dermique :

- HISPAGEL :
   (commercialisé par la Société Hispanochimica et constitué de polyméthacrylate de glycérol). 20
- Esters d'acide parahydroxybenzoïque : 0,20
- 3,5-di-t-butyl-4-hydroxybenzoate :
   d'octaoxyéthylèneglycol 2
- Eau déminéralisée : QSP 100 g

### Gouttes buvables :

- 3,5-di-t-butyl-4-hydroxybenzoate :
   d'octaoxyéthylèneglycol 2,50
- Alcoolature de citron et d'orange : 0,70
- Glycérine : 10
- Paraoxybenzoate de méthyles sodé : 0,15
- Gallate de propyle : 0,001
- Eau purifiée : QSP 100 g

### Soluté injectable en intramusculaire :

- 3,5-di-t-butyl-4-hydroxybenzoate :
   d'octaoxyéthylèneglycol 2,50
- Sulfite monosodique : 0,10
- Citrate de sodium : 1
- Paraoxybenzoate de méthyle : 0,13
- Paraoxybenzoate de propyle : 0,02
- Eau pour préparation injectable : QSP 100 g

### Comprimé :

- 3,5-di-t-butyl-4-hydroxybenzoate :
   d'octaoxyéthylèneglycol 2,50
- Lactose : 5
- Amidon de maïs : 10
- Silice colloïdale : 10
- Stéarate de magnésium : 5
- Gélatine : 1
- Talc QSP 100 g

### III - ACTIVITÉS DES COMPOSÉS DE FORMULE (I) A L'ENCONTRE DES VIRUS DE L'HERPÈS.

On a apprécié les propriétés virucides du 3,5-di-t-butyl-4-hydroxybenzoate d'octaoxyéthylèneglycol de formule : 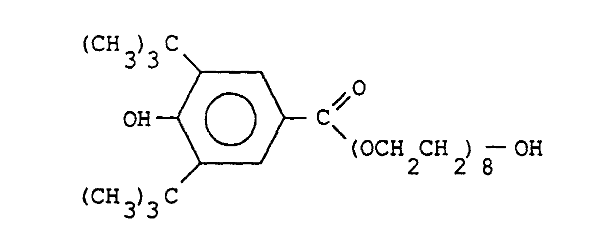 dénommé ci-après par commodité AVF₁, sur un échantillon de "virus-stock" de souche HSV₁. Le titre infectieux (TV) d'échantillons traités avec AVF₁ a été comparé au titre infectieux (TV₀) d'un échantillon témoin afin de calculer la valeur log (TV/TV₀).

Le protocole a consisté à incuber à 37°C l'échantillon de virus HSV₁ stock avec le composé AVF₁, en faisant varier la concentration en AVF1 et le temps d'incubation. TV₀ a été déterminé à partir d'une composition constituée de 0,5 ml de milieu de culture MEM et 0,5 ml de virus-stock et TV a été déterminée à partir d'une composition constituée de 0,5 ml de AVF1 à diverses concentrations dans ledit milieu MEM et de 0,5 ml de virus-stock. Les valeurs TV₀ et TV obtenus sont des multiples de la DI₅₀/ml.

### 1) Essai 1 :

- Concentrations en AVF1 : 0,03 ; 0,06 ; 0,12 ; 0,25 ; 0,5 ; 1,25 ; 2,5 ; 5 mg/ml.
- Temps d'incubation : 60 et 120 minutes.
- Résultats :
   Titre du témoin-virus : 2 x 106 DI₅₀/ml.
   AVF₁ < 2,5 mg/ml : TV.
   AVF₁ ≥ 2,5 mg/ml : 0.
- Conclusions :
   AVF₁ est virucide en 60 minutes à la concentration 2,5 mg/ml.

### 2) Essai 2 :

- Concentrations en AVF1 : 1 et 5 mg/ml.
- Temps d'incubation : 5, 15, 30, et 60 minutes.
- Résultats :
   Titre du témoin-virus : 2 x 10⁶ DI₅₀/ml
   AVF₁ = 5 mg/ml : virucide en 5 minutes, titre = 0.
   AVF₁ = 1 mg/ml : le titre est réduit de 1,5 log 10 en 60 minutes.

### IV - ACTIVITÉS DES COMPOSÉS DE FORMULE (I) A L'ENCONTRE DES VIRUS HIV.

On a apprécié les propriétés anti-VIH-1 du 3,5-di-t-butyl-4-hydroxybenzoate d'octaoxyéthylèneglycol de formule : 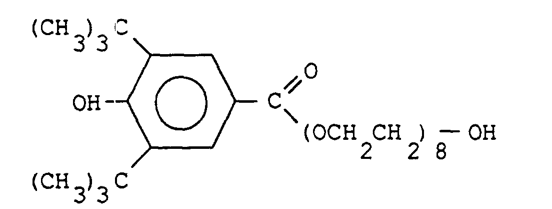 dénommé ci-après par commodité AVF₁.

### 1) Principe.

Le Virus de l'Immunodéfience Humaine type 1 (VIH-1) appartient à la famille des Rétroviridiae, sous famille des lentivirinae, genre Lentivirus (Field B. and Knipe D. in "Fields Virology (Vol. 1 and 2) Raven Press Ltd, 1990 ; Vogt P. The oncovirinae. A definition of the group. in report n°1 of WHO collaborating centre for collection and evaluation of data on comparative virology. Munich : UNI-Druck, 1976, p. 327-339). Le virus est enveloppé et son diamètre est de 80 à 130 nm. Son génome est un dimère d'AAV de polarité positive de 9,2 kb. C'est un virus pertinent pour l'étude de l'efficacité de procédés destinés à la désinfection d'instruments ou de surfaces potentiellement contaminés par des agents pathogènes pour l'homme. Il est peu résistant aux traitements physico-chimiques.

Afin d'évaluer l'efficacité d'AVF1, il a été procédé à la comparaison des titres infectieux de suspensions virales avant et après traitement par trois concentrations de AVF1 pendant trois temps de contact. A partir des résultats expérimentaux, il a été défini pour chaque concentration et chaque temps de contact testés, un facteur de reproduction du titre infectieux du VIH-1. Ce facteur de réduction exprimé en log 10 correspond au rapport du titre infectieux du virus non traité et du titre infectieux du virus traité par AVF1.

### 2) Matériel.

### a) Les cellules:

Les lignées cellulaires suivantes ont été utilisées pour l'étude (Wain-Hobson S. et al. *Science*, 1991, 252, 961-965 ; Foley G. et al. *Cancer*, 1965, 18, 522-529) :
- Cellules CEM (ATCC CCL-119) (McDougal J. et al. *J. Immunol.,* 1985, 135, 3151-3162 ; Harada S. et al. *Science,* 1985, 229, 563-566) : lignee de cellules lymphoblastoïdes T humaines établie à partir du sang périphérique d'un patient atteint d'une leucémie lymphoblastique aigüe. Les cellules sont cultivées dans du milieu RPMI 1640 (Whittaker) additionné de 10% de sérum de veau foetal (Gibco), 1% de glutamine et 1% de penicilline/streptomycine/néomycine. Elles sont utilisées pour produire la souche virale VIH-1/LAI.
- Cellules MT4 (Rey F. et al. *J. Virol. Meth.*, 1987, 16, 239-249 ; Barré-Sinoussi F. et al. *Science,* 1983, 220, 868-871) : cette lignée de cellules T humaines transformées par le HTLV-1 est cultivée dans le milieu suivant : RPMI 1640 (Whittaker) additionné de 10% de sérum de veau foetal (Gibco), 1% d'antibiotique (PSN, Gibco) et 1% de glutamine (Gibco). Ces cellules sont utilisées pour le titrage du VIH-1.

### b) Le virus :

La souche de virus VIH-1/LAI (Barré-Sinoussi F. et al. *Science*, 1983, 220, 868-871 ; Rey M. et al. *Biochem. Biophys. Res. Comm*., 1984, 121, 126-133 ; Gregersen J. et al. *J. Virol. Meth.*, 1988, 19, 161-168 ; Hoffman A. et al. *Virology,* 1985, 147, 326-335) a été utilisée pour cette étude. Le VIH-1 (souche LAI) est produit sur une lignée de cellules lymphoblastoïdes T chroniquement infectées (lignée CEM/VIH-1/LAI). Tous les 3 ou 4 jours, les cellules sont centrifugées, remises en suspension dans du milieu de culture et mélangées à 50% avec des cellules CEM non infectées afin de maintenir la production virale. Après clarification des surnageants de culture à 2000 rpm pendant 20 minutes, le virus présent dans ces surnageants est concentré par centrifugation pendant 3 heures à 17000 rpm. Le culot est repris en tampon NTE (NaCl 0,1 M ; Tris 0,01 M ; EDTA 0,001 ; pH 7,4), aliquoté et stocké à -80°C. Le virus utilisé pour cette étude a un titre infectieux supérieur à 10⁶ "Tissue Culture Infectious Dose 50" par ml (TCID50/ml) et un titre en activité transcriptase inverse supérieur à 10⁶ cpm/ml.

### 3) Méthode.

L'étude a été réalisée en deux étapes :
- Traitement du VIH-1/LAI par trois concentrations d'AVF1 pendant 5, 30 et 60 minutes.
- Evaluation des titres infectieux des échantillons obtenus avant et après traitement par AVF1 sur cellules MT4.

### a) Traitement du VIH-1 :

Le virus a été dilué au 1/10ème dans chacune des solutions de AVF1 (solution diluée à 2%, 1%, et 0,5%).

Le virus a été maintenu dans chacune de ces solutions pendant 5 minutes, 30 minutes et 60 minutes, à température ambiante puis immédiatement dilué en tampon NTE. Après dilution en tampn NTE, les échantillons ont été ultracentrifugés afin d'éliminer AVF1 et éviter ainsi sa toxicité sur les cellules MT4 utilisées pour le titrage du virus. Cette étape d'ultracentrifugation permet aussi de concentrer les particules virales résiduelles. Après centrifugation, chaque culot a été repris dans 1 ml de milieu de culture, filtré sur un filtre de 0,45 µm aliquoté et stocké à -80°C.

La même expérience a été réalisée deux fois.

### b) Plusieurs contrôles ont été réalisés pour chaque expérience :

- Contrôles négatifs :
   . Les cellules utilisées pour le titrage, non infectées (culture témoin).
   . les trois concentrations de AVF1 utilisées dans l'étude, diluées en tampon NTE et ultracentrifugées. Ces échantillons (contrôles négatifs) ont été utilisés pour vérifier l'absence de toxicité due à des traces résiduelles de AVF1, sur les cellules MT4.
- Contrôles positifs :
   . Le VIH-1 dilué en milieu de culture à la dilution réalisée pour l'étude, immédiatement aliquoté et stocké à -80°C (contrôle positif).
   . Le VIH-1 dilué en milieu de culture à la dilution réalisée pour l'étude, puis dilué en tampon NTE, ultracentrifugé, filtré sur un filtre de 0,45 µm, aliquoté, stocké à -80°C et titré ultérieurement (contrôle de centrifugation).
   . Le VIH-1 dilué en milieu de culture à la dilution réalisée pour l'étude, laissé 10 minutes à température ambiante, puis dilué en tampon NTE, ultracentrifugé, filtré sur un filtre de 0,45 µm, aliquoté, stocké à -80°C et titré ultérieurement (contrôle expérimental).

### 4) Titrage.

### a) Principe :

La méthode de titrage du VIH-1 sur cellules MT4 est basée sur la détection d'une production virale par les cellules inoculées maintenues 10 jours en culture, mesurée par le dosage de l'antigène p24 VIH-1 dans le surnageant de culture.

Brièvement, les cellules MT4 sont inoculées avec des dilutions en série de virus non traité (contrôle positif) ou avec les échantillons expérimentaux (virus traités par AVF1).

Le VIH infecte les cellules, se multiplie, puis est libéré dans le surnageant de culture par bourgeonnement à la surface de la cellule. Afin de détecter la présence du virus, les surnageants de culture sont récupérés tous les 3 ou 4 jours et testés au jour +10 après l'infection quant à la présence de l'antigène p24 VIH-1.

La détection de l'antigène p24 VIH-1 est réalisée à l'aide d'une technique ELISA sandwich mettant en oeuvre la trousse ELAVIA AgI commercialisée par Diagnostic Pasteur, qui permet la détection de 50 pg d'antigène p24 par millilitre de surnageant.

Le principe de cette méthode est brièvement le suivant : l'antigène p24 libéré après la lyse des particules virales présentes dans le surnageant est complexé à des anticorps anti-VIH-1 immobilisés dans les puits des microplaques. L'antigène est alors révélé par anticorps polyclonaux anti-VIH-1 biotinylés, reconnus eux-mêmes par un conjugué streptavidine péroxydase. La présence de ce complexe est révélée par une réaction enzymatique après addition du substrat (H₂O₂) et du chromogène (OPD). Le produit de cette réaction est coloré et peut donc être détecté par mesure de la densité optique à 492 nm. La quantité de produit formé au cours de la réaction enzymatique est directement proportionnelle à la quantité d'antigène présente dans l'échantillon testé.

### b) Métode de titrage sur cellules MT4 :

Les échantillons à tester ont été dilués ou non dans du milieu de culture. 1 ml d'échantillon non dilué, et 0,4 ml des dilutions suivantes ont été inoculés sur un culot de 1,2 10⁶ cellules MT4. Après l'adsoption virale pendant 1 heure à 37°C, l'innoculum viral a été éliminé et les cellules lavées 3 fois en milieu de culture. Les cellules ont alors été resuspendues dans 4 ml de milieu de culture, distribuées dans 4 puits de plaque 24 puits et incubées à 37°C en présence de 5% de CO₂.

Tous les 3 ou 4 jours, les surnageants de culture contenus dans chaque puits ont été prélevés et stockés à -80°C puis testés quant à la présence d'antigène p24 VIH-1.

### c) Définition du titre infectieux sur cellules MT4 :

Le titre infectieux est exprimé en "tissue Culture Infectious Dose 50" par millilitre d'échantillon (TCID 50/ml). Il correspond à l'inverse de la dernière dilution qui induit une production virale dans 2 puits sur 4. Cette production virale est détectée par dosage de l'antigène p24 dans les surnageants des cellules MT4, au jour +10 après l'inoculation.

### d) Cytotoxicité :

Le contrôle de toxicité a été réalisé dans des conditions identiques à celles décrites pour la méthode de titrage, mais en inoculant les cellules MT4 avec les échantillons non contaminés par le VIH-1 et décrits précédemment (contrôle négatif).

Le critère de non toxicité du produit est établi a été établi en comparant l'aspect morphologique et la viabilité des cellules MT4 inoculées avec du milieu de culture (culture témoin) ou avec les échantillons non contaminés (contrôles négatifs) après 10 jours de culture.

### e) Limite de détection :

La limite de détection de la méthode de titrage dépend de la sensibilité du test et de la dilution minimale non toxique utilisée pour inoculer les cellules. La valeur seuil du test de détection de l'antigène est déterminé pour chaque échantillon en comparant les valeurs obtenues dans un surnageant de culture témoin non infecté et dans le surnageant testé.

Donc, lorsqu'aucune toxicité n'a été observée avec l'échantillon non dilué, inoculé sous un volume de 250 µl sur les cellules MT4 et que les surnageants du jour 10 après l'inoculation sont négatifs en antigène p24, la limite de détection du titrage est de 4 TCID 50/ml.

### 5) Résultats.

Les résultats de l'étude sont rapportés dans les tableaux I et II ci-après, indiquant respectivement les titres infectieux des échantillons témoins et des échantillons expérimentaux.

Les titres infectieux calculés d'après la méthode de Spearman et Karber, correspondent à l'inverse de la dilution qui induit une production virale dans 50% des puits inoculés. La production virale est détectée par dosage de l'antigène p24 VIH-1 dans les surnageants des cultures au jour +10 après infection.

**Tableau I**

| | Echantillons (1 par expérience) | Titre TCID 50/ml | | Remarques |
|---|---|---|---|---|
| | | 1ère expérience | 2ème expérience | |
| Témoins Négatifs | AVF1 dilué à 2%, non contaminé, dilué et centrifugé | - | - | Non toxique (échantillon inoculé à la dilution 1/4) |
| | AVF1 dilué à 1%, non contaminé, dilué et centrifugé | - | - | Non toxique (échantillon inoculé à la dilution 1/4) |
| | AVF1 dilué à 0,5%, non contaminé, dilué et centrifugé | - | - | Non toxique (échantillon inoculé à la dilution 1/4) |
| Témoins Positifs | Virus à la dilution utilisée pour l'expérimentation | > 10^{7,5} | > 10^{7,5} | Témoin positif |
| | Virus à la dilution utilisée pour l'expérimentation puis centrifugé et filtré | > 10^{7,5} | > 10^{7,5} | Aucune perte d'infectivité due à la centrifugation et la filtration |
| | Virus à la dilution utilisée pour l'expérimentation, laissé à température ambiante avant ultracentrifugation et filtration | > 10^{7,5} | > 10^{7,5} | Aucune perte d'infectivité due aux conditions expérimentales |

**Tableau II**

| Echantillons (1 par expérience) | Titre TCID 50/ml | | Facteur de réduction Log TCID50/ml | |
|---|---|---|---|---|
| | 1ère expérience | 2ème expérience | 1ère expérience | 2ème expérience |
| VIH-1 traité par AVF1 à 2% pendant 5 minutes | < 4 | < 4 | > 6,9 | > 6,9 |
| VIH-1 traité par AVF1 à 2% pendant 30 minutes | < 4 | < 4 | > 6,9 | > 6,9 |
| VIH-1 traité par AVF1 à 2% pendant 60 minutes | < 4 | < 4 | > 6,9 | > 6,9 |
| VIH-1 traité par AVF1 à 1% pendant 5 minutes | < 4 | < 4 | > 6,9 | > 6,9 |
| VIH-1 traité par AVF1 à 1% pendant 30 minutes | < 4 | < 4 | > 6,9 | > 6,9 |
| VIH-1 traité par AVF1 à 1% pendant 60 minutes | < 4 | < 4 | > 6,9 | > 6,9 |
| VIH-1 traité par AVF1 à 0,5% pendant 5 minutes | < 4 | < 4 | > 6,9 | > 6,9 |
| VIH-1 traité par AVF1 à 0,5% pendant 30 minutes | < 4 | < 4 | > 6,9 | > 6,9 |
| VIH-1 traité par AVF1 à 0,5% pendant 60 minutes | < 4 | < 4 | > 6,9 | > 6,9 |

### a) Echantillons témoins :

- Aucune toxicité n'a été observée avec les échantillons contrôles négatifs inoculés à la dilution 1/4 sur les cellules MT4 (Tableau I)
- Aucune diminution du titre infectieux n'a été observée pour les échantillons témoins utilisés pour évaluer la perte de virus due à l'étape de centrifugation et de filtration, et due aux conditions expérimentales. En effet, les titres infectieux des échantillons témoins sont :
   . supérieur ou égal à 10^{7,5} TCID50/ml pour les témoins positifs (Tableau I);
   . supérieur ou égal à 10^{7,5} TCID50/ml pour les témoins de centrifugation et filtration (Tableau I),
   . supérieur ou égal à 10^{7,5} TCID50/ml pour les témoins de conditions expérimentales.

### b) Echantillons expérimentaux :

Aucune particule infectieuse VIH-1 n'a été détectée dans les échantillons de virus traité par AVF1, et ce, quelque soit la concentration et le temps de contact (Tableau II). Comme le montre le tableau II, les surnageants des cultures inoculées avec des dilutions 1/4 des échantillons de virus traités se sont révélés négatifs en antigène p24 VIH-1, ce qui indique qu'après traitement, les virions présents dans les inoculums sont incapables de se répliquer dans les cellules MT4.

Selon la limite de détection du titrage, le titre infectieux des échantillons expérimentaux est donc < 4 TCID50/ml dans les deux espériences réalisées.

### 6) Conclusion.

Cette étude montre que le traitement par AVF1 est efficace pour inactiver le VIH-1. Selon les titres infectieux évalués sur cellules MT4, le traitement du virus par une solution à 0,5% de AVF1 pendant 5 minutes, induit une réduction du pouvoir infectieux supérieure ou égale à 6,9 log TCID50/ml.

### V - ACTIVITÉS DES COMPOSÉS DE FORMULE (I) SUR LE SYSTEME INDUCTIBLE DE LA SYNTHASE DE MONOXIDE D'AZOTE.

On a apprécié l'activité sur le système inductible de la synthase de monoxide d'azote du 3,5-di-t-butyl-4-hydroxybenzoate d'octaoxyéthylèneglycol de formule, dénommé ci-après par commodité AVF1.

### 1) Introduction.

Le monoxyde d'azote (NO) est dégagé dans les cellules lors de la transformation de la L-arginine en L-citruline. Ce radical libre hautement instable est immédiatement métabolisé en nombreux composés intermédiaires du cycle de l'azote (anion superoxide O₂-, nitrites -NO₂₋- et nitates -NO₃₋-). L'enzyme responsable de la synthèse du NO est une NO synthase dont il existe quatre formes, 2 constitutives et 2 inductibles iNOS (Berdeaux A. *Fundam. Clin. Pharmacol.,* 1993, 7(8), 401-411) dont une est présente dans les macrophages activés sous l'effet d'un agent extérieur notamment microbien. Le rôle antimicrobien et inflammatoire du monoxyde d'azote (NO) et de ses dérivés est maintenant bien établi (Chao et al., *Clin. Immunol. Immunopath.*, 1993, 68, 178-183 ; Croen K. D., *J. Clin. Invest.*, 1993, 91, 2446-2452 ; Gunasagaran et al., *Science,* 1993, 261, 1445-1448).

AVF1 a un pouvoir anti-oxydant qui pourrait agir via le système de la iNOS. A la faveur d'une infection ce principe actif pourrait concourir à la réduction des nitrates en nitrites ou en d'autres dérivés intermédiaires hautement toxiques voir induire lui-même l'isoforme macrophagique de la NOS comme cela a été décrit pour des extraits de plantes par Greenspan H. C. and Aruoma O. K.

Cette hypothèse a été contrôlée en dosant les nitrites dans des surnageants de culture de macrophages murins de lignées activées en présence de AVF1. Les nitrites ont été dosés par la réaction de Griess après réduction de tous le nitrates grâce à la nitrate réductase de Pseudomonas oleoverans (Granger et al., J. Immunol., 1991, 146, 1294-1302). La spécificité de la réaction a été prouvée par l'emploi d'un inhibiteur spécifique de la synthèse du monoxyde d'azote, la N^{G}-monométhyl-L-arginine (L-NMMA).

### 2) Méthode.

### a) Essai de toxicité de AVF1 :

La toxicité de AVF1 pour les macrophages de lignée RAW264.7 (ATCC TIB 71) utilisés pour le test iNOS a été déterminée dans l'expérience préliminaire suivante :

Les macrophages de lignée RAW264.7 ont été distribués dans des séries de 4 puits dans une plaque à 96 puits à raison de 1,5 x 10⁵/100 µl/ml dans du milieu de culture (D-NEM sans rouge de phénol, Gibco) additionné de 0,2 % d'antibiotique, 1% de L-glutamine et 5% de sérum de veau foetal décomplémenté (produits pour celuture cellulaire Eurobio).

Après 18 heures, différentes concentrations de raison 2 de AVF1 ont été ajoutées sous 100 µl de façon à obtenir les concentrations finales de 10⁰ à 10⁻¹⁰%. Après 24 heures de culture supplémentaire, les cellules ont été observées au microscope et colorées selon deux techniques différentes permettant d'obtenir un pourcentage respectivement de cellules adhérentes au plastique (coloration au violet cristal) et de cellules vivantes (coloration au rouge neutre) :
- Dans la première technique, les cellules sont lavées avec du PBS + Ca²⁺ + Mg²⁺ (Gibco) afin d'éliminer les éventuelles cellules mortes flottantes et de maintenir les cellules vivantes sur le plastique. Le colorant (1% de violet cristal, R.A.L. 20% de méthanol, Merck) est ajouté sous 50 µl/puits et laissé 20 minutes à température ambiante. Après plusieurs lavages doux en PBS + Ca²⁺ + Mg²⁺ , les plaques sont séchées à 37°C et 100 µl de SDS (Sigma, 1% en eau distillée) sont ajoutés par puits. La plaque est laissée 1 heure à température ambiante à l'abri de la lumière pour permettre la dissolution complète du lysat cellulaire. La plaque est ensuite lue au lecteur ELISA (Labsystems) à 550 nm après 15 secondes d'agitation. Le blanc de densité optique (OD) est fait sur une rangée de 8 cupules n'ayant reçu que 50 µl de la solution de violet cristal puis ayant été traitées comme les autres.
- Dans la seconde technique, les cellules reçoivent 50 µl de rouge neutre à 0,3% (R.A.L.) qui est pinocyté par les macrophages vivants. Après 2 heures d'incubation, le milieu est enlevé et les cellules sont lavées doucement avec 200 µl de PBS + Ca²⁺ + Mg²⁺. Les cellules restantes sont ensuite lysées avec 100 µl de SDS et la plaque est mise à 4°C pendant 1 heure pour permettre un relargage complet du colorant. La plaque est ensuite lue au lecteur ELISA à 570 nm avec un filtre de référence de 630 nm après 15 secondes d'agitation. Le blanc de DO est fait sur une colonne de 8 cupules n'ayant reçu que le colorant puis ayant été traitées comme les autres.

Pour la réalisation de cet essai, AVF1 a été préparé de la façon suivante : 1 g a été dissout dans 5 ml de D-MEM sans rouge de phénol. Cette solution à 20% a été stérilisée sur 0,22 µm (Minisart, Sartorius), le pH contrôlé (7,5), aliquotée sous 1 ml et conservée à +4°C. Au moment de l'emploi, cette solution a été diluée afin d'obtenir les concentrations finales désirées.

### b) Essai d'induction du système iNOS :

Le test d'induction iNOS consiste à ensemencer les macrophages RAW264.7 dans des plaques à 24 puits à raison de 1 x 10⁶ sous 1,6 ml. Après 24 heures de culture permettant d'obtenir un tapis cellulaire complet, AVF1, dilué de raison 10 dans des des doses ne donnant pas de toxicité cellulaire (de 10⁻⁶ à 10⁻⁸ %), est ajouté dans 100 µl par cupule soit sur des macrophages sans activateur, avec ou sans l'inhibiteur de iNOS (action directe du produit sur les macrophages) soit en présence des inducteurs, avec ou sans inhibiteur (action du produit sur des macrophages activés).

Après 24 heures de culture, les surnageants sont prélevés, centrifugés 10 minutes à 100 g à température ambiantes pour éliminer les éventuels débris cellulaires puis utilisés pour le dosage des nitrites.

### c) Essai de dosage des nitrites par la réaction de Griess après réduction des nitrates par la nitrate réductase :

Deux volumes des surnageants clarifiés sont réduits en présence d'un volume d'une suspension de Pseudomonas oleovorans à 100 mg/ml et un volume de D-MEM sans rouge de phénol. Après une incubation de 90 minutes à 37°C sans agitation, les mélanges sont centrifugés 5 minutes à 4 000 g pour éliminer les bactéries et dilués au 1/5 et au 1/10 en eau distillée stérile.

100 µl des échantillons réduits et dilués sont distribués dans des séries de 2 puits dans une plaque ELISA (maxicorp, Nunc.). 200 µl du réactif de Griess (mélange ex-temporané volume à volume des solutions de sulfamide à 1,5 g/100 ml de HCl 1,2 N (87,1 mM) - réactif A - et de naphtylènediamine à 0,15 g/100 ml d'eau stérile (5,78 mM) - réactif B -) sont ajoutés.

Cent autres µl d'échantillon servant de blanc de DO sont distribués et mélangés à 200 µl d'un mélange ex-temporané volume à volume d'eau distillée et du réactif colorimétrique A.

Après 30 minutes à température ambiante à l'obscurité, la densité optique (DO) des échantillons est lue à 540 nm au lecteur de plaque pour ELISA.

### d) Contrôle des essais :

- Les contrôles du système d'induction iNOS sont composés de :
   . Témoin cellules consistant en des macrophages ne recevant que du milieu de culture.
   . Témoin d'induction consistant en des macrophages traités par 100 µl de lipopolysaccharide (LPS à 4 µg/ml, Difco) et par 100 µl d'interféron-γ recombinant (IFN-γ à 1 µU/ml, Boerhinger-Mannheim) afin d'activer la iNOS (Schmidt et al., *Mol. Pharmacol.,* 1992, 41, 614-624). Les solutions mères de LPS et de IFN-γont été conservées respectivement à +4°C et à -20°C avant utilisation.
   . Témoin d'inhibition spécifique pour prouver que les nitrites dosés proviennent de l'activation de la iNOS. Il consiste à incorporer à la culture un inhibiteur spécifique, la L-NMMA (N^{G}-monométhyl-L-arginine, Calbiochem) (Hibbs et al., *Science,* 1987, 235, 473-476) à des concentrations de raison 10 allant de 3 à 300 µM. Cette solution a été conservée sous parties aliquotes à -20°C avant utilisation.
- Les contrôles de la réaction de Greiss sont composés de :
   . Une solution de nitrate de sodium qui permet de contrôler l'activité nitrate-réductase des bactéries.
   . Une gamme étalon de nitrite de sodium qui permet de doser les nitrites dans les échantillons.
- Le blanc de DO de la gamme étalon contenant 100 µl d'eau distillée et 200 µl du mélange des réactifs A et B.

### 3) Résultats.

### a) Toxicité de AVF1 pour les macrophages RAW 264.7 :

**Tableau III**

| VIOLET CRISTAL | | | |
|---|---|---|---|
| | | DO/ml moyenne sur 4 puis ± écart type | Pourcentage de survie par rapport au témoin cellules en milieu seul |
| Milieu | | 2,088 ± 0,013 | 100 |
| AVF1 | 1 % | 0,172 ± 0,030 | 8 |
| | 0,5 % | 0,118 ± 0,027 | 6 |
| | 1 10⁻² % | 0,118 ± 0,027 | 6 |
| | 5 10⁻³ % | 0,061 ± 0,017 | 3 |
| | 1 10⁻³ % | 0,117 ± 0,027 | 8 |
| | 5 10⁻⁴ % | 0,156 ± 0,010 | 7 |
| | 1 10⁻⁴ % | 0,180 ± 0,016 | 9 |
| | 5 10⁻⁵ % | 0,216 ± 0,034 | 10 |
| | 1 10⁻⁵ % | 0,259 ± 0,033 | 12 |
| | 5 10⁻⁶ % | 1,129 ± 0,069 | 54 |

**Tableau IV**

| ROUGE NEUTRE | | | |
|---|---|---|---|
| | | DO/ml moyenne sur 4 puis ± écart type | Pourcentage de survie par rapport au témoin cellules en milieu seul |
| Milieu | | 0,682 ± 0,037 | 100 |
| AVF1 | 1 % | 0 | 0 |
| | 0,5 % | 0 | 0 |
| | 1 10⁻² % | 0 | 6 |
| | 5 10⁻³ % | 0 | 3 |
| | 1 10⁻³ % | 0 | 0 |
| | 5 10⁻⁴ % | 0 | 0 |
| | 1 10⁻⁴ % | 0,010 ± 0,002 | 1 |
| | 5 10⁻⁵ % | 0,023 ± 0,003 | 16 |
| | 1 10⁻⁵ % | 0,060 ± 0,034 | 41 |
| | 5 10⁻⁶ % | 0,476 ± 0,034 | 70 |

AVF1 est toxique jusqu'à la concentration de 5 x 10⁻⁵ %. Une autre expérience préliminaire de toxicité a donc été réalisée avec des concentrations plus faibles, dont les résultats sont rapportés dans les tableaux V et VI ci-dessous.

**Tableau V**

| VIOLET CRISTAL | | | |
|---|---|---|---|
| | | DO/ml moyenne sur 4 puis ± écart type | Pourcentage de survie par rapport au témoin cellules en milieu seul |
| Milieu | | 1,113 ± 0,067 | 100 |
| AVF1 | 10⁻⁶ % | 0,991 ± 0,078 | 89 |
| | 5 10⁻⁷ % | 1,051 ± 0,041 | 94 |
| | 1 10⁻⁷ % | 0,960 ± 0,070 | 86 |
| | 5 10⁻⁸ % | 1,089 ± 0,051 | 98 |
| | 1 10⁻⁸ % | 1,128 ± 0,043 | 100 |
| | 5 10⁻⁹ % | 0,970 ± 0,062 | 87 |
| | 1 10⁻⁹ % | 1,010 ± 0,059 | 91 |
| | 5 10⁻¹⁰ % | 1,000 ± 0,089 | 90 |
| | 1 10⁻¹⁰ % | 0,934 ± 0,050 | 84 |

**Tableau VI**

| ROUGE NEUTRE | | | |
|---|---|---|---|
| | | DO/ml moyenne sur 4 puis ± écart type | Pourcentage de survie par rapport au témoin cellules en milieu seul |
| Milieu | | 0,635 ± 0,042 | 100 |
| AVF1 | 10⁻⁶ % | 0,602 ± 0,059 | 95 |
| | 5 10⁻⁷ % | 0,652 ± 0,089 | 100 |
| | 1 10⁻⁷ % | 0,605 ± 0,024 | 95 |
| | 5 10⁻⁸ % | 0,575 ± 0,040 | 90 |
| | 1 10⁻⁸ % | 0,601 ± 0,121 | 95 |
| | 5 10⁻⁹ % | 0,545 ± 0,015 | 86 |
| | 1 10⁻⁹ % | 0,594 ± 0,083 | 93 |
| | 5 10⁻¹⁰ % | 0,573 ± 0,030 | 90 |
| | 1 10⁻¹⁰ % | 0,609 ± 0,053 | 96 |

AVF1 n'est plus toxique sur des macrophages RAW 264.7 après 24 heures de culture à partir de 10⁻⁶ %. C'est donc les concentrations à partir de 10⁻⁶ % qui ont été utilisées pour les expériences d'induction du monoxyde d'azote.

### b) Induction du système iNOS :

Le tableau VII ci-dessous rapporte les résultat de la production de nitrites dans les puits traités par AVF1.

**Tableau VII**

| Traitement des cellules | Milieu | AVF1 | | |
|---|---|---|---|---|
| | | 1 x 10⁻⁶ % (10 nM) | 1 x 10⁻⁷ % (1 nM) | 1 x 10⁻⁸ % (0,1 nM) |
| Milieu | 5,3* | 5,0* | 4,8* | 5,6* |
| IFNγ + LPS | 20,8* | 20,9* (0 %)** | 19,5* (6 %)** | 21,6* (0 %)** |
| IFNγ + LPS + L-NMMA (3 µM) | 20,9* (0 %)** | 5,3* (74 %)** | 5,1* (75 %)** | 5,0* (76 %)** |
| IFNγ + LPS + L-NMMA (30 µM) | 14,7* (29 %)** | 6,3* (70 %)** | 4,9* (76 %)** | 5,2* (75 %)** |
| IFNγ + LPS + L-NMMA (300 µM) | 9,3* (55 %)** | 4,8* (77 %)** | 5,8* (72 %)** | 5,5* (74 %)** |

| | | | | |
|---|---|---|---|---|
| * : Concentration moyenne en nitrites en µM sur 2 échantillons. | | | | |
| ** : Pourcentage d'inhibition de la production de nitrite. | | | | |

### 4) Conclusion.

AVF1 est hautement toxique sur les macrophages murins de la lignée jusqu'à 10⁻⁵ %, soit environ 100 nM.

A partir de 10⁻⁶ %, AVF1 n'induit pas de monoxyde d'azote (NO) dans les macrophages, dosé par la présence de nitrites dans les surnageants de culture.

Lorsque ces macrophages sont activés par la combinaison de LPS et d'IFN-γ pour induire la production de NO via la NO-synthase inductible, AVF1 n'a aucune action sur le niveau de production.

AVF1 aux doses utilisées n'a donc aucune action directe sur le système inductible de la NO synthase macrophagique.

Lorsque les macrophages activés sont traités par la N^{G}-monométhyl-L-arginine (LNMMA), inhibiteur spécifique de la synthèse de NO, AVF1 augmente l'action de cet inhibiteur. Ainsi à une concentration non inhibitrice de L-NMMA (3 µM), 75 % de la production de NO par les macrophages est inhibée.

En conséquence, AVF1 agit à très faible concentration en synergie avec un inhibiteur de la NO-synthase inductible. Cette propriété confère au composé de l'Invention un intérêt tout particulier pour traiter, en association avec un inhibiteur de la NO-synthase inductible comme le L-NMMA, les maladies dans lesquelles le monoxyde d'azote à une action pathologique, tels que l'inflammation, la constriction vasculaire ou les cancers.

Cette propriété est en outre particulièrement intéressante, car l'effet anti-virale des composés de l'Invention et notamment de AVF1, utiles dans le traitement du SIDA, est renforcée par les propriétés anti-inflammatoires montrées ci-dessus sur la réplication des virus HIV.

## Revendications

1. Les composés de formule : 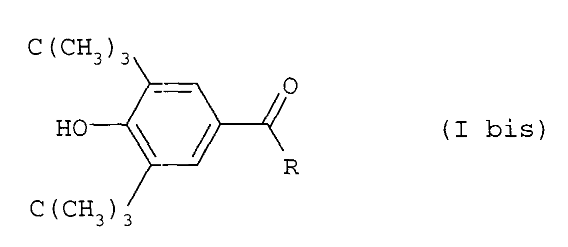 dans laquelle le radical R est choisi parmi les 3 groupements de formules suivantes :
―(OCH₂CH₂)ₙ―Y (II)
―O(CH₂CH₂NH)ₙ―Y (III) 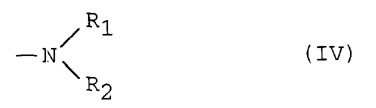
dans lesquelles :
. n est un nombre entier compris entre 1 et 20; mais on peut tout à fait envisager une valeur maximale de n de 80 ;
. R1 est choisi parmi les groupes suivants:
- (CH₂CH₂O)ₙ-Y,-(CH₂CH₂NH)ₙ-Y ;
. R2 est un atome d'hydrogène ou a la même signification que R1 ; et
. Y est choisi parmi les groupes suivants: un atome d'hydrogène, un groupe -OH, un groupe -NH2, un groupe -COOH, un groupe 3,5-di-t-butyl-4-hydroxybenzoate, un groupe amine ou hydroxyamine primaire, secondaire ou tertiaire, un groupe amide, diamide, hydroxyamide ou halogénoamide aliphatique ou aromatique, un groupe alkoyle en C₁-C₁₂, un groupe glycoyle, un groupe glycéryle mono-, di- ou tri-substitué, un groupe hétérocyclique, un groupe thioester aliphatique ou aromatique, un groupe de formule : -COOZ, où Z est un groupe alkyle en C₁-C₁₂, un groupe alcényle en C₁-C₁₂, un groupe alcynyle en C₁-C₁₂, un groupe hydroxyalkyle en C₁-C₁₂, un groupe hydroxyalcynyle en C₁-C₁₂, un groupe aminoalkyle en C₁-C₁₂, un groupe aminoalcényle en C₁-C₁₂. un groupe aryl, un groupe hétérocyclique.

2. Les composés de formule (I bis) dans laquelle le radical R représente un groupement de formule :
―(OCH₂CH₂)ₙ―Y (II)
―O(CH₂CH₂NH)ₙ―Y (III)
dans lesquelles, Y et n ont la même signification que dans la revendication 1.

3. Les composés de formule (I bis) dans laquelle le radical R représente un groupement de formule : 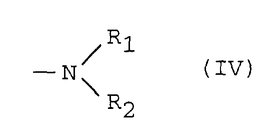 dans laquelle, R₁ et R₂ ont la même signification que dans la revendication 1.

4. Le 3,5-di-t-butyl-4-hydroxybenzoate d'octaoxyéthylèneglycol de formule :

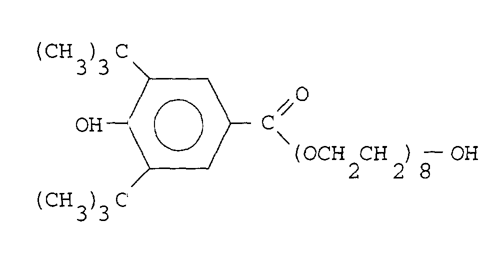

5. Les composés selon l'une quelconque des revendications 1 à 3, **caractérisés en ce que** dans les formules (II), (III) et (IV), Y représente un groupe de formule :

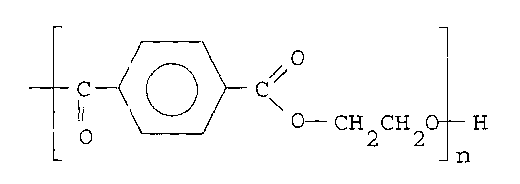

6. Composition pharmaceutique **caractérisée en ce qu**'elle renferme à titre de principe actif au moins un composé selon l'une quelconque des revendications 1 à 5, éventuellement en association avec un véhicule pharmaceutiquement acceptable.

7. Composition pharmaceutique selon la revendicaton 6, **caractérisée en ce qu**'elle renferme à titre de principe actif au moins un composé de formule (I) : 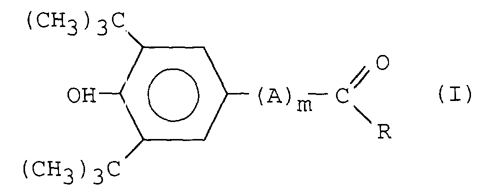 dans laquelle :
- A est un groupe -CH₂-,
- m est un nombre entier compris entre 0 et 12, et
- le radical R est choisi parmi les trois groupements de formules :
―(OCH₂CH₂)ₙ―Y (II)
―O(CH₂CH₂NH)ₙ―Y (III) 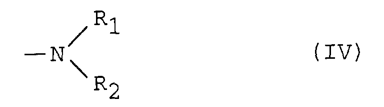
dans lesquelles, n, R1, R2 et Y ont la même signification que dans la revendication 1.

8. Composition pharmaceutique selon la revendication 7, **caractérisée en ce qu**'elle renferme à titre de principe actif le 3,5-di-t-butyl-4-hydroxybenzoate d'octa-oxy-éthylèneglycol

9. Composition pharmaceutique selon l'une des revendications 6 à 8, **caractérisée en ce qu**'elle renferme en outre un inhibiteur de la NO-synthase inductible comme le L-NMMA.

10. Utilisation des composés de formule (I) tels que définis dans la revendication 7, pour l'obtention d'un médicament destiné au traitement et à la prévention chez l'homme et l'animal d'une infection par les virus.

11. Utilisation selon la revendication 10, **caractérisée en ce que** le médicament est destiné au traitement et à la prévention d'une infection par les virus à enveloppe lipidique.

12. Utilisation selon la revendication 10, **caractérisée en ce que** le médicament est destiné au traitement et à la prévention d'une infection par les virus de l'herpès et HIV.

13. Utilisation selon la revendication 10, **caractérisée en ce que** le médicament est destiné au traitement et à la prévention d'une infection par les papillomavirus.

14. Utilisation selon la revendication 10, **caractérisée en ce que** le médicament est destiné au traitement des condylomes notamment ano-génitaux, dus aux papillomavirus humains.

15. Utilisation des composés de formule (I) tels que définis dans la revendication 7 en association avec un inhibiteur de la NO-synthase inductible comme le L-NMMA, pour l'obtention d'un médicament destiné au traitement des maladies dans lesquelles le monoxyde d'azote a une action pathologique.

16. Utilisation des composés de formule (I) tels que définis dans la revendication 7 pour renforcer les propriétés antiseptiques, antivirales, antibactériennes et antifongiques des tissus pharmaceutiques du type pansement, bandage ou compresse ou d'implants.

17. Utilisation selon la revendication 16, caractérisée qu'au moins une partie des fibres dudit tissu pharmaceutique ou implant est greffée par au moins un composé de formule (I) tel que défini à la revendication 7.

18. Tissu pharmaceutique du type pansement, bandage ou compresse ou implant, **caractérisé en ce qu**'au moins un composé de formule (I) tel que défini à la revendication 7, est greffé sur tout ou partie des fibres constituant ledit tissu pharmaceutique.

19. Tissu pharmaceutique ou implant selon la revendication 18, **caractérisé en ce qu**'au moins une partie des fibres qui le constituent comprend le composé de la revendication 4.

## Patentansprüche

1. Verbindungen der Formel: 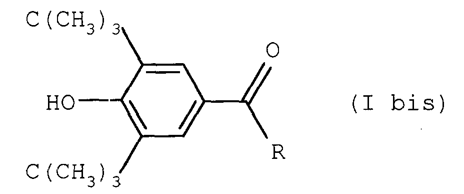 in welcher der Rest R aus den drei Gruppen mit den folgenden Formeln gewählt wird:
-(OCH₂CH₂)ₙ-Y (II)
-O(CH₂CH₂NH)ₙ-Y (III) 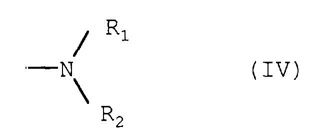
in denen:
. n eine ganze Zahl zwischen 1 und 20 ist, aber auch ein Maximalwert von 80 ist durchaus möglich;
. R1 gewählt wird unter den folgenden Gruppen:
-(CH₂CH₂O)ₙ-Y, - (CH₂CH₂NH)ₙ-Y;
. R2 ein Wasserstoffatom ist oder dieselbe Bedeutung hat wie R1; und
. Y unter den folgenden Gruppen ausgewählt wird: ein Wasserstoffatom, eine OH-Gruppe, eine NH-Gruppe, eine COOH-Gruppe, eine 3,5-Di-tertiärbutyl-4-hydroxybenzoat-Gruppe, eine primäre, sekundäre oder tertiäre Amino- oder Hydroxyaminogruppe, eine aliphatische oder aromatische Amid-, Diamid-, Hydroxyamid- oder Halogenamidgruppe, eine Alkoholgruppe C₁-C₁₂, eine Glykolgruppe, eine mono- di- oder trisubstituierte Glycerolgruppe, eine heterozyklische Gruppe, eine aliphatische oder aromatische Thioestergruppe, eine Gruppe der Formel -COOZ, wobei Z ein Alkylrest C₁-C₁₂, ein Alkenrest C₁-C₁₂, ein Alkinrest C₁-C₁₂, ein Hydroxyalkylrest C₁-C₁₂, ein Hydroxyalkenrest C₁-C₁₂, ein Hydroxyalkinrest C₁-C₁₂, ein Aminoalkylrest C₁-C₁₂, ein Aminoalkenrest C₁-C₁₂, ein Arylrest, ein heterozyklischer Rest ist.

2. Die Verbindungen der Formel (Ib) in denen der Rest R eine Gruppe der Formel
-(OCH₂CH₂)ₙ-Y (II)
-O(CH₂CH₂NH)ₙ-Y (III)
darstellt, wobei Y und n die gleiche Bedeutung haben wie in Anspruch 1.

3. Die Verbindungen der Formel (Ib) in denen der Rest R eine Gruppe der Formel 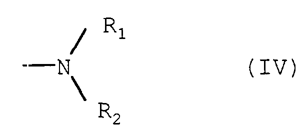 darstellt, wobei R1 und R2 die gleiche Bedeutung haben wie in Anspruch 1.

4. Octaoxyethylenglycol-3,5-di-tertiärbutyl-4-hydroxybenzoat mit der Formel:

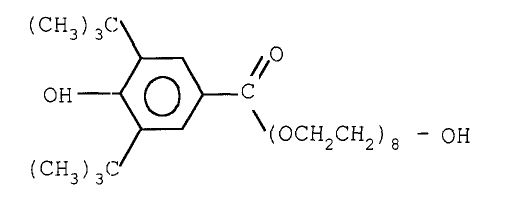

5. Die Verbindungen nach irgendeinem der Ansprüche 1 bis 3, die **dadurch gekennzeichnet** werden, daß in den Formeln (II), (III) und (IV), Y eine Gruppe der Formel

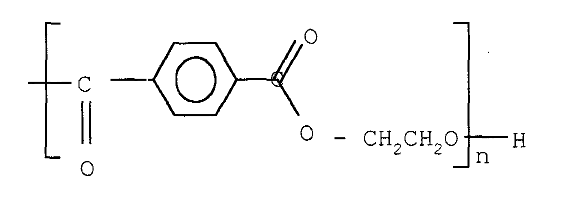

6. Pharmazeutische Zubereitung, die **dadurch gekennzeichnet** wird, daß sie als Wirkstoff mindestens eine Verbindung nach irgendeinem der Ansprüche 1 bis 5 enthält, eventuell in Verbindung mit einem pharmazeutisch akzeptablen Arzneistoffträger.

7. Pharmazeutische Zubereitung nach Anspruch 6, die **dadurch gekennzeichnet** wird, daß sie als Wirkstoff mindestens eine Verbindung der Formel (I) : 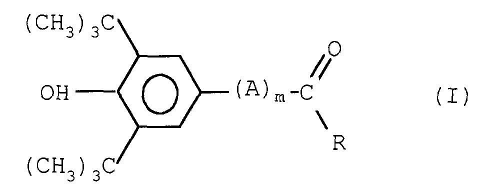 enthält, in der:
- A eine -CH₂-Gruppe ist,
- m eine Ganze Zahl zwischen 0 und 12 ist
- der Rest R aus den drei Gruppen mit den folgenden Formeln gewählt wird:
-(OCH₂CH₂)ₙ-Y (II)
-O(CH₂CH₂NH)ₙ-Y (III) 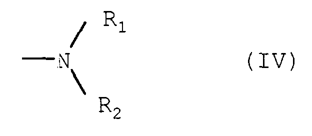
in denen n, R1, R2 und Y die gleiche Bedeutung haben wie in Anspruch 1.

8. Pharmazeutische Zubereitung nach Anspruch 7, die **dadurch gekennzeichnet** wird daß sie als Wirkstoff Octaoxyethylenglycol-3,5-di-tertiärbutyl-4-hydroxybenzoat enthält.

9. Pharmazeutische Zubereitung nach einem der Ansprüche 6 bis 8, die **dadurch gekennzeichnet** wird, daß sie zusätzlich einen induktiblen NO-Synthase-Inhibitor wie L-NMMA enthält.

10. Anwendung der Verbindungen der Formel (1) wie in Anspruch 7 definiert um ein Medikament zu erhalten das zur Behandlung und Vorbeugung von Virusinfektionen von Menschen und Tieren dient.

11. Anwendung nach Anspruch 10, die **dadurch gekennzeichnet** wird, daß das Medikament zur Behandlung und Vorbeugung einer Infektionen mit Viren mit Lipidhülle dient.

12. Anwendung nach Anspruch 10, die **dadurch gekennzeichnet** wird, daß das Medikament zur Behandlung und Vorbeugung einer Infektionen mit Herpes- und HIV-Viren dient.

13. Anwendung nach Anspruch 10, die **dadurch gekennzeichnet** wird, daß das Medikament zur Behandlung und Vorbeugung einer Infektionen mit Papilloma-Viren dient.

14. Anwendung nach Anspruch 10, die **dadurch gekennzeichnet** wird, daß das Medikament zur Behandlung von -insbesondere ano-genitalen- Condylomen dient, die durch menschliche Papillomaviren hervorgerufen werden.

15. Anwendung der Verbindungen der Formel (I) wie in Anspruch 7 definiert in Verbindung mit einem induktiblen NO-Synthase-Inhibitor wie L-NMMA, um ein Medikament zu erhalten das der Behandlung von Krankheiten dient, bei denen das Stickstoff-Monoxyd eine pathologische Wirkung hat.

16. Anwendung der Verbindungen der Formel (I) wie in Anspruch 7 definiert, zur Verstärkung der antiseptischen, antiviralen, antibakteriellen und antimykotischen Eigenschaften von pharmazeutischen Geweben wie Pflastern, Verbänden oder Kompressen, oder von Implantaten.

17. Anwendung nach Anspruch 16, die **dadurch gekennzeichnet** wird, daß zumindest ein Teil der Fasern des erwähnten pharmazeutischen Gewebes oder Implantats mit mindestens einer Verbindung der Formel (I) wie in Anspruch 7 definiert behandelt wird.

18. Pharmazeutisches Gewebe wie Pflaster, Verband oder Kompresse oder Implantat, das **dadurch gekennzeichnet** wird, daß mindestens eine Verbindung der Formel (I) wie in Anspruch 7 definiert auf alle oder einen Teil der Fasern des erwähnten Gewebes aufgebracht wird.

19. Pharmazeutisches Gewebe oder Implantat nach Anspruch 18, das **dadurch gekennzeichnet** wird, daß mindestens ein Teil der Fasern, aus dem es hergestellt ist, die Verbindung aus Anspruch 4 enthalten.

## Claims

1. Compounds of the formula: 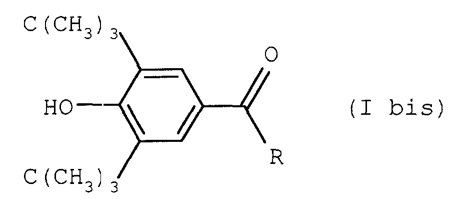 in which the radical R is chosen from among the 3 following formula groupings:
― (OCH₂CH₂)ₙ ― Y (II)
― O(CH₂CH₂NH)ₙ ― Y (III) 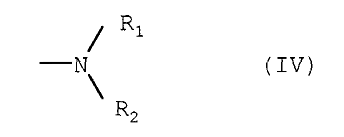
in which:
. n is a whole number comprised between 1 and 20; but a maximum value for n of 80 can be perfectly well envisaged;
. R1 is chosen from among the following groups:
- (CH₂CH₂O)ₙ-Y, - (CH₂CH₂NH)ₙ-Y ;
. R2 is an atom of hydrogen or has the same significance as R1; and
. Y is chosen from among the following groups: a hydrogen atom, an -OH group, an -NH₂ group, a -COOH group, a 3,5-di-t-butyl-4-hydroxybenzoate group, an amine or primary, secondary or tertiary hydroxyamine group, an aliphatic or aromatic amide, diamide, hydroxyamide or halogenamide group, an alkoyl C₁-C₁₂ group, a glycoyl group, a mono-, di-- or tri-substituted glyceryl group, a heterocyclic group, an aliphatic or aromatic thioester group, a group with formula: -COOZ, where Z is a C₁-C₁₂ alkyl group, a C₁-C₁₂ alkenyl group, a C₁-C₁₂ alkynyl group, a C₁-C₁₂ hydroxyalkyl group, a C₁-C₁₂ hydroxyalkynyl group, a C₁-C₁₂ aminoalkyl group, a C₁-C₁₂ aminoalkenyl group, an aryl group, a heterocyclic group.

2. Compounds of formula (I bis) in which the radical R represents a grouping of formula:
― (OCH₂CH₂)ₙ ― Y (II)
― O(CH₂CH₂NH)ₙ ― Y (III)
in which Y and n have the same significance as in claim 1.

3. Compounds of formula (I bis) in which the radical R represents a grouping of formula: 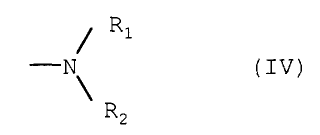 in which R1 and R2 have the same significance as in claim 1.

4. The 3,5-di-t-butyl-4-hydroxybenzoate of octa-oxy-ethylene glycol of formula:

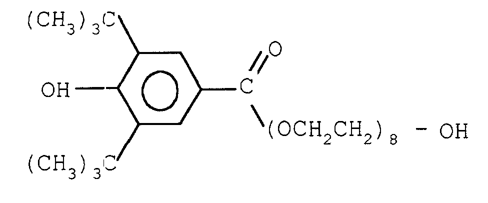

5. Compounds according to any one of claims 1 to 3, **characterised in that** in the formulae (II), (III) and (IV), Y represents a group of formula:

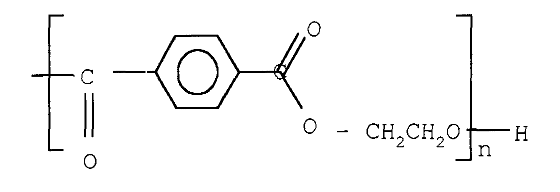

6. Pharmaceutical composition **characterised in that** it includes as its principle active agent at least one compound according to any one of claims 1 to 5, possibly in association with a suitable pharmaceutical excipient.

7. Pharmaceutical composition according to claim 6, **characterised in that** it includes as its principle active agent at least one compound of formula (I): 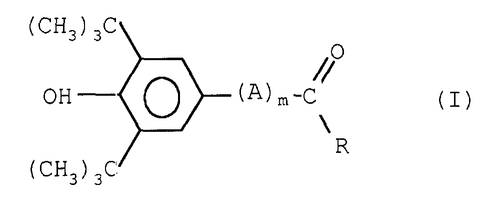 in which:
- A is a -CH2- group,
- m is a whole number comprised between 0 and 12, and
- the radical R is chosen among the three formula groupings:
― (OCH₂CH₂)ₙ ― Y (II)
― O(CH₂CH₂NH)ₙ ― Y (III) 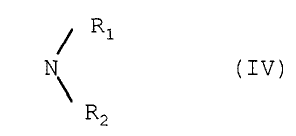
in which, n, R1, R2 and Y have the same significance as in claim 1.

8. Pharmaceutical composition according to claim 7 **characterised in that** it includes 3,5-di-t-butyl-4-hydroxy-benzoate of octa-oxy-ethylene glycol as its principle active agent.

9. Pharmaceutical composition according to any one of claims 6 to 8, **characterised in that** it also includes an inhibitor of inducible NO-synthase such as L-NMMA.

10. Utilisation of compounds of formula (I) such as defined in claim 7, for obtaining a drug intended for the treatment and prevention in man and animal of an infection by viruses.

11. Utilisation according to claim 10, **characterised in that** the drug is intended for the treatment and prevention of an infection by viruses with a lipidic envelope.

12. Utilisation according to claim 10, **characterised in that** the drug is intended for the treatment and prevention of an infection by the herpes and HIV viruses.

13. Utilisation according to claim 10, **characterised in that** the drug is intended for the treatment and prevention of an infection by the papillomaviruses.

14. Utilisation according to claim 10, **characterised in that** the drug is intended for the treatment of condylomas in particular genito-anal, due to human pappilomaviruses.

15. Utilisation of compounds of formula (I) such as defined in claim 7 in association with an inhibitor of inducible NO-synthase such as L-NMMA, for obtaining a drug intended for treating illnesses in which nitrogen monoxide has a pathological action.

16. Utilisation of compounds of formula (I) such as defined in claim 7 for reinforcing the antiseptic, antiviral, antibacterial and fungicidal properties of pharmaceutical fabrics of the dressing, bandage or compress type or of implants.

17. Utilisation according to claim 16, **characterised in that** at least one part of the fibres of said pharmaceutical fabric or implant is grafted by at least one compound of formula (I) as defined in claim 7.

18. Pharmaceutical fabric of the dressing, bandage or compress type or implant, **characterised in that** at least one compound of formula (I) such as defined in claim 7, is grafted on all or part of the fibres constituting said pharmaceutical fabric.

19. Pharmaceutical fabric or implant according to claim 18, **characterised in that** at least one part of the fibres constituting it comprises the compound of claim 4.
